# EUROPEAN PATENT SPECIFICATION

(11) **EP 4 309 667 B1**
(45) Date of publication and mention of the grant of the patent: **04.09.2024**
(21) Application number: 22185497.9
(22) Date of filing: 18.07.2022
(51) Int. Cl.: A61K 38/30, A61K 9/08, A61K 35/16, A61P 27/02, A61K 38/18, A61K 9/00, A61K 47/30

(54) **MULTI-DONOR HUMAN SERUM PRODUCT, METHOD FOR MANUFACTURE, AND ITS USE IN TREATMENT OF DRY EYE DISEASE**
MULTI-DONOR PRODUKT VON HUMANSERUM, VERFAHREN ZU DESSEN HERSTELLUNG UND VERWENDUNG ZUR BEHANDLUNG DER KRANKHEIT DES TROCKENEN AUGES
PRODUIT SERUM HUMAIN MULTI-DONNERUS, SON PROCÉDÉ DE FABRICATION, ET UTILISATION POUR LE TRAITEMENT DE LA MALADIE DE L' OEIL SEC

(43) Date of publication of application: 24.01.2024
(73) Proprietor: Sanaplas GmbH, 30559 Hannover (DE)
(72) Inventor: Jochheim-Richter, Andrea, 30559 Hannover (DE)
(74) Representative: Fuchs Patentanwälte Partnerschaft mbB

(56) References cited:
- WO-A1-2022/029109
- DE-A1- 102009 022 793
- US-B2- 10 300 088
- GEERLING G ET AL: "[Autologous serum-eye-drops for ocular surface disorders. A literature review and recommendations for their application] AUTOLOGE SERUM-AUGENTROPFEN ZUR THERAPIE DER AUGENOBERFLAECHE", OPHTHALMOLOGE, SPRINGER, BERLIN, DE, vol. 99, no. 12, 1 December 2002 (2002-12-01), pages 949 - 959, XP002397834, ISSN: 0941-293X, DOI: 10.1007/S00347-002-0661-6
- J [ \ZIK\: "Quality Control Analytical Methods", INTERNATIONAL JOURNAL OF PHARMACEUTICAL COMPOUNDING, 1 January 2009 (2009-01-01), XP055271988, Retrieved from the Internet <URL:http://www.arlok.com/articles/Applications and Sterility of Autologous Serum Eye Drops.pdf> [retrieved on 20160511]
- LEE P.D.K. ET AL: "High molecular weight forms of insulin-like growth factor II and its binding protein identified by protein immunoblotting", BIOCHEMICAL AND BIOPHYSICAL RESEARCH COMMUNICATIONS, vol. 152, no. 3, 1 May 1988 (1988-05-01), Amsterdam NL, pages 1131 - 1137, XP093007152, ISSN: 0006-291X, DOI: 10.1016/S0006-291X(88)80402-9
- HINTZ RAYMOND L.: "2 Plasma forms of somatomedin and the binding protein phenomenon", CLINICS IN ENDOCRINOLOGY AND METABOLISM, vol. 13, no. 1, 1 March 1984 (1984-03-01), US, pages 31 - 42, XP093055045, ISSN: 0300-595X, DOI: 10.1016/S0300-595X(84)80007-9
- DE VROEDE M A ET AL: "Modulation of insulinlike growth factor I binding to human fibroblast monolayer cultures by insulinlike growth factor carrier proteins released to the incubation media.", THE JOURNAL OF CLINICAL INVESTIGATION, vol. 77, no. 2, 1 February 1986 (1986-02-01), GB, pages 602 - 613, XP093055048, ISSN: 0021-9738, Retrieved from the Internet <URL:https://www.jci.org/articles/view/112343/version/1/pdf/render.pdf> DOI: 10.1172/JCI112343
- SMITH ET AL: "Somatomedin carrier proteins", MOLECULAR AND CELLULAR ENDOCRINOLOGY, ELSEVIER IRELAND LTD, IE, vol. 34, no. 2, 1 February 1984 (1984-02-01), pages 83 - 89, XP025224568, ISSN: 0303-7207, [retrieved on 19840201], DOI: 10.1016/0303-7207(84)90058-3

## Description

The invention relates to a product suitable for the treatment of tear film deficiency and regeneration of corneal epithelia in dry eye disease and a method for manufacturing the product. The invention also relates to a product for use in medicine, as well as a product for use in the treatment of dry eye disease. The product according to the invention provides for an improved wound healing in dry eye disease and other pathologies.

### Background

A lack in the aqueous phase of the tear film is a main causative factor that leads to the prime pathology of tear film break-up in dry eye disease. Oxidative stress occurs as result of the disruption of the balance between the antioxidant system and the pro-oxidant system, damages the ocular surface and plays a critical role in dry eye disease due to increased oxidized protein levels in accordance with the elevated inflammatory activity during this disease. In addition, lack of corneal epithelial integrity leads to water influx into the stroma.

Serum has found numerous applications in diagnosis, and has also been used in medical therapies. Serum refers to the soluble components of whole blood which is deprived of the clotting factors and all types of cells, for example by coagulation of a blood sample and subsequent centrifugation and filtration. Serum thus includes essentially all proteins not active in blood clotting, in addition to electrolytes, antibodies, antigens, and hormones.

Tear fluid in healthy subjects contains several antioxidants, such as ascorbic acid, lactoferrin, uric acid and cysteine, to protect the ocular surface against certain radicals. It has been speculated that the components present in human serum may provide for an antioxidant effect that could be harnessed for the treatment of tear film deficiency in dry eye disease.

WO 2022/029109 A1 describes compositions and biologically active blood fractions for treating damaged epithelial surfaces in connection with eye diseases. WO 2022/029109 A1 also describes corresponding methods of production, i.e. the fractions are obtained via ultracentrifugation and subsequent separation from total serum or total plasma. The obtained blood fractions are formulated into eye drops. The described fractions of blood obtained after serum ultracentrifugation were found to be biologically active and to have a healing effect on damaged epithelial cells. The obtained serum fractions were substantially free of lipids and lipo-proteins which was found to increase their flow ability facilitating bottling of the fractions and sterile filtration for the removal of pathogens, such as bacteria.

The healthy cornea has a limited water influx (leak) into the stroma which is counter-balanced by water efflux due to hydrogen carbonate, lactate and NaCl solutes across the De-scemet layer and the endothelial monolayer. Oxidative stress occurs as a result of the tear break-up followed by disruption of the balance between the anti-oxidant system and the pro-oxidant system and damages the ocular surface and plays a critical role in dry eye disease due to increased oxidized protein levels in accordance with the elevated inflammatory activity during these diseases. In addition, lack of corneal epithelial integrity leads to water influx into the stroma. The described condition needs to be treated adequately and efficiently, in particular epithelial defects need to be closed quickly and the tear film stabilized, in order to avoid long-term damage and worsening of dry eye disease.

However, the pharmaceutical products known in the art do not provide a sufficient regenerative capacity, i.e. by healing corneal epithelial defects. Accordingly, there is an unmet therapeutic need for novel products that are more active against the dry eye disease and related pathologies.

The above objects are solved by the subject-matter of the claims and by the subject-matter described here below.

### Summary of the invention

In a first aspect, the invention relates to a multi-donor human serum product comprising insulin-like growth factors (IGFs) bound in macromolecular complexes of a molecular size of 30 kDa or more, the product comprising 20 ng/ml IGF-1 or more, the product comprising less than 70 mg/dl monosaccharides, wherein the product is optionally an isotonic solution or an isotonic gel.

In a related aspect, the invention relates to a human serum product comprising insulin-like growth factors (IGFs) bound in macromolecular complexes of a molecular size of 30 kDa or more, the product comprising 20 to 100 ng/ml IGF-1, the product comprising less than 70 mg/dl monosaccharides, wherein the product is optionally an isotonic solution or an isotonic gel.

The inventors have discovered that human serum comprises insulin-like growth factors (IGFs) bound in macromolecular complexes of a molecular size of at least 30 kDa, and that these macromolecular complexes provide for an improved wound healing when they are left intact and incorporated into a pharmaceutical product. Human serum contains specific epitheliotrophic factors such as IGF-1, EGF, and others, in addition to a high concentration of proteins such as albumin and fibronectin. Serum directly supports the proliferation and migration of epithelial cells or indirectly enhances epithelial viability by binding and neutralising inflammatory cytokines. Growth factors and cytokines are important regulators that stimulate growth, proliferation, migration, differentiation, adhesion, as well as ECM deposition and proteinase regulation of cells involved in wound healing.

In a second aspect, the invention relates to the human serum product for use in medicine.

In a related aspect, the invention relates to the human serum product for use in the treatment of dry eye disease.

In a third aspect, the invention relates to a method for manufacturing a human serum product, comprising the steps of
- providing human allogeneic blood volume portions from a multitude of donors,
- selecting volume portions from at least two donors,
- deriving serum from the selected volume portions to obtain serum portions,
- pooling the selected serum portions to obtain pooled serum, wherein optionally the selected serum portions are from donors all having an age of 45 years or less;
- obtaining an intermediate product from the pooled serum, the intermediate product comprising 100 ng/ml IGF-1 or more; and
- obtaining the human serum product according to this disclosure from the intermediate product.

The method relies on providing whole blood volume portions from a multitude of donors which are selected in such a way to ensure a sufficient IGF concentration. IGF-1 concentrations in human blood differ between individuals. Some volume portions may have very high IGF, others very low IGF-1 concentrations. By selecting volume portions with adequate IGF-1 concentrations, a desired IGF-1 concentration in the product can be obtained. For example, it was found that the IGF-1 concentration in human serum decreases with an increasing lifetime of the donors. Hence, restricting the donor age of the multitude of donors to 45 years or less contributes to an efficient production method as less blood has to be discarded. The method provides for obtaining an intermediate product, wherein the intermediate product comprises 100 ng/ml IGF-1 or more. The obtained product comprises insulin-like growth factors (IGFs) bound in macromolecular complexes of a molecular size of 30 kDa or more. Advantageously, the method is versatile with respect to choosing variants, parameters and conditions to obtain the product according to the invention from the intermediate product, and thus provides a product which has improved therapeutic properties, e.g. for repairing corneal epithelial defects and anti-inflammation properties.

In an aspect not according to the claimed invention, this disclosure provides an intermediate product comprising insulin-like growth factors (IGFs) bound in macromolecular complexes of a molecular size of 30 kDa or more, wherein the intermediate product comprises 100 to 500 ng/ml IGF-1.

### Brief description of the drawings

**Figure 1A** and **Figure 1B****,** respectively, show the age-dependant IGF-1 and IGF-BP3 concentrations in human serum. The age is given in years.
**Figures 2A** and **2B** show the activity of serum fractions in an *in vitro* wound closure assay.
**Figures 3A** to **3D****,** respectively, show the effect of four different human recombinant (hr) growth factors, i.e. EGF, IGF-1, PDGF and KGF, on wound closure when combined with the high-molecular weight fraction or the low-molecular weight fraction of ultra-filtered multi-donor human serum, respectively.
**Figure 4** shows the anti-inflammatory effect of three high-molecular weight fraction samples in a (LPS)-TNF-α inhibition assay.

### Detailed description

### Product

In a first aspect, the invention relates to a multi-donor human serum product comprising insulin-like growth factors (IGFs) bound in macromolecular complexes of a molecular size of 30 kDa or more, the product comprising 20 ng/ml IGF-1 or more, the product comprising less than 70 mg/dl monosaccharides, wherein the product is optionally an isotonic solution or an isotonic gel.

In a related aspect, the invention relates to a human serum product comprising insulin-like growth factors (IGFs) bound in macromolecular complexes of a molecular size of 30 kDa or more, the product comprising 20 to 100 ng/ml IGF-1, the product comprising less than 70 mg/dl monosaccharides, wherein the product is optionally an isotonic solution or an isotonic gel.

If this disclosure refers to a 'human serum product', it refers equally to the 'multi-donor human serum product' as well as the 'human serum product'.

Human serum comprises several growth factors, e.g. insulin-like growth factors, specifically insulin-like growth factor 1 (IGF-1) and insulin-like growth factor 2 (IGF-2), as well as the binding protein IGF-BP3 and ALS, all of which can be experimentally quantified. The inventors have discovered that human serum comprises insulin-like growth factors bound in macromolecular complexes of a molecular size of more than 30 kDa, and that these macromolecular complexes provide for an improved wound healing when they are left intact and incorporated into a pharmaceutical product. IGF-1 and IGF-2 have a molecular weight of, respectively, 7.65 kDa and 7.5 kDa (referring to the human proteins). The macromolecular complexes comprise IGF-1 and/or IGF-2 together with their binding proteins and thus attain a molecular size of 30 kDa or more. It has been established and/or confirmed that the macromolecular complexes are left intact in the absence of proteases and at fairly neutral pH values, i.e. pH around 7. High-salt conditions, or addition of organic solvents, or addition of protease inhibitors would destroy the macromolecular complexes.

In one embodiment, the macromolecular complexes have a molecular size of 40 to 50 kDa. In one embodiment, the macromolecular complexes have a molecular size of up to 150 kDa.

IGFs bound in macromolecular complexes also provide for a constant availability of active growth factors and improves the storage stability of the product. IGFs in their monomeric form are more prone to degradation which is thought to result from the lack of their native form.

In one embodiment, the human serum product comprises less than 70 mg/dl monosaccharides, less than 40 mg/dl monosaccharides, or less than 30 mg/dl monosaccharides. Advantageously, the human serum product comprises IGFs bound in macromolecular complexes which have been found to accelerate wound healing, combined with a low amount of low-molecular components typically found in human serum, i.e. monosaccharides. For example, by way of reference, whole blood of healthy donors comprises 60 to 140 mg/dl (< 5.6 to 7.8 mmol/l) blood glucose level. Low-molecular components are generally accepted to have a molecular weight below 900 Dalton. Low-molecular component concentrations in serum are for example depleted in a retentate obtained after ultracentrifugation, or in a human serum product obtained by diluting human serum.

In one embodiment, the human serum product comprises less than 40 mg/dl total cholesterol, wherein total cholesterol is the sum of LDL (low-density lipoprotein) and HDL (high-density lipoprotein). Advantageously, the human serum product comprises IGFs bound in macromolecular complexes which have been found to accelerate wound healing, combined with a certain amount of further high-molecular components typically found in human serum, i.e. cholesterol. For example, by way of reference, whole blood of healthy donors comprises about 150 mg/dl total cholesterol. High-molecular components in serum are enriched at least to some extent with respect to their concentration in a retentate obtained after ultracentrifugation. In a human serum product obtained by diluting human serum, the concentrations of these high-molecular components are diluted respectively.

In one embodiment, the human serum product comprises IGFs bound in macromolecular complexes of a molecular size of 30 kDa or more, the product comprising 20 ng/ml IGF-1 or more, and comprises less than 70 mg/dl monosaccharides, less than 55 mg/dl monosaccharides, less than 40 mg/dl monosaccharides, less than 30 mg/dl monosaccharides, less than 20 mg/dl monosaccharides, or less than 10 mg/dl monosaccharides.

In one embodiment, the human serum product comprises IGFs bound in macromolecular complexes of a molecular size of 30 kDa or more, the product comprising 20 to 100 ng/ml IGF-1, and comprises less than 70 mg/dl monosaccharides, less than 55 mg/dl monosaccharides, less than 40 mg/dl monosaccharides, less than 30 mg/dl monosaccharides, less than 20 mg/dl monosaccharides, or less than 10 mg/dl monosaccharides.

In the context of this disclosure, total cholesterol and urea are constituents of human serum which do not serve or contribute to a specific function of the human serum product. Provided that whole blood is obtained from healthy donors, the contents of total cholesterol and urea in the human serum product are not considered harmful.

In one embodiment, the human serum product comprises less than 40 mg/dl total cholesterol, wherein total cholesterol is the sum of LDL (low-density lipoprotein) and HDL (high-density lipoprotein), less than 35 mg/dl total cholesterol, or less than 30 mg/dl total cholesterol. In one embodiment, the product comprises 5 mg/dl total cholesterol or more, 10 mg/dl total cholesterol or more, or 15 mg/dl total cholesterol or more. In one embodiment, the product comprises 5 to 40 mg/dl total cholesterol, 10 to 35 mg/dl total cholesterol, or 15 to 30 mg/dl total cholesterol.

In one embodiment, the human serum product comprises less than 70 mg/dl LDL and/or less than 30 mg/dl HDL. In one embodiment, the product comprises 2 to 70 mg/dl LDL and/or 1 to 30 mg/dl HDL. In one embodiment, the product comprises 2 to 70 mg/dl LDL, 5 to 60 mg/dl LDL, 10 to 50 mg/dl LDL, or 15 to 40 mg/dl LDL. In one embodiment, the product comprises 1 to 30 mg/dl HDL, 2 to 25 mg/dl HDL, or 5 to 20 mg/dl HDL.

In one embodiment, the human serum product comprises less than 100 mg/dL urea, or less than 40 mg/dL urea, or less than 10 mg/dL urea, or less than 3 mg/dL urea.

The human serum product may be derived from human serum, and is preferably derived from human allogeneic blood. The human serum product can hence be easily manufactured and requires only a limited number and amount of additives. In one embodiment, the human serum product comprises ingredients and/or additives, such as e.g. vitamins, growth factors, pH modifiers, buffering agents, surfactants, viscosity modifiers, and/or comprises NaCl in an amount so that the product is isotonic with respect to its intended pharmaceutical application.

In one embodiment, the human serum product is essentially free of stabilisers. In one embodiment, the human serum product is essentially free of preserving agents. The skilled person knows that stabilisers or preserving agents, if desired, need to be deliberately added. The requirements that the human serum product is essentially free of stabilisers, as well as the requirements that the human serum product is essentially free of preserving agents, means that neither stabilisers are deliberately added nor that preserving agents are deliberately added to the human serum product. Whereas typical pharmaceutical products may require preserving agents, such as e.g. benzalkonium chloride (which is an irritant to the eyes), the human serum products according to this disclosure work in the absence of such agents. The intermediate product obtained from the human allogeneic blood is, thanks to its way of manufacture, not prone to microbial contamination, and can thus be manufactured, further processed and stored in the absence of preserving agents.

In one embodiment, the human serum product is an isotonic solution or an isotonic gel. In the context of the invention, the human serum product has pharmaceutical applications and is used in pharmacy and/or medicine. In particular for the treatment of eye-related conditions, the tonicity or osmolarity of the human serum product has to be provided and/or maintained at physiological levels. The skilled person thus understands that the reference to an isotonic solution or an isotonic gel means that the effective osmolar concentration of the human serum product is essentially the same as that of relevant bodily fluids. The solute concentration of the human serum product is thus adjusted, to become an isotonic solution or an isotonic gel, with respect to the solutes concentration inside human cells or human tissue.

Osmolality refers to the concentration of osmotically active solutes in a solution, whereas tonicity refers to the corresponding physiological effect in the body, also termed "effective osmolality".

In one embodiment, the human serum product has a pH value between 5.6 and 10.5, 7.3 to 9.7, or about 7.4.

In one embodiment, the human serum product has a tonicity of 127 to 475 mosmol/kg, 222 to 445 mosmol/kg, 250 to 320 mosmol/kg, or about 285 mosmol/kg.

In one embodiment, the human serum product has a pH value between 5.6 and 10.5, and a tonicity of 127 to 475 mosmol/kg. In one embodiment, the human serum product has a pH value between 7.3 to 9.7, and a tonicity of 222 to 445 mosmol/kg. In one embodiment, the human serum product has a pH value of about 7.4, and a tonicity of about 285 mosmol/kg. A pH value of about 7.4, and a tonicity of about 285 mosmol/kg are generally considered physiological concentrations, thus providing for a good physiological tolerance of an ophthalmic solution.

In one embodiment, the human serum product comprises IGF-1 at a concentration of 20 ng/ml or more, 30 ng/ml or more, 50 ng/ml or more, or 100 ng/ml or more. In one embodiment, the human serum product comprises IGF-1 at a concentration of 600 ng/ml or less, 500 ng/ml or less, 400 ng/ml or less, or 300 ng/ml or less. In one embodiment, the human serum product comprises IGF-1 at a concentration of 20 to 600 ng/ml, 30 to 500 ng/ml, 50 to 400 ng/ml, or 100 to 300 ng/ml. The presence of IGF-1 in the human serum product contributes to the wound healing properties of the product.

Insulin-like growth factor-binding protein 3 (IGF-BP3) is a protein encoded by the *hIGF8P3* gene. IGF-BP3 is one of six IGF-binding proteins (IGF-BP to IGF-BP6) which bind the insulin-like growth factors IGF-1 and IGF-2 with high affinity. IGFBP-3 functions in the blood circulation, in the extracellular environment, and inside cells. It is considered the main IGF transport protein in the bloodstream, where it carries the growth factors predominantly in stable complexes that contain the binding protein, either IGF-1 or IGF-2, and a third protein called the acid-labile subunit (ALS).

In one embodiment, the human serum product comprises 1300 ng/ml IGF-BP3 or more, 2000 ng/ml IGF-BP3 or more, or 3000 ng/ml IGF-BP3 or more. In one embodiment, the human serum product comprises 8000 ng/ml IGF-BP3 or less, 7000 ng/ml IGF-BP3 or less, or 6000 ng/ml IGF-BP3 or less. In one embodiment, the product comprises 1300 to 8000 ng/ml IGF-BP3, 2000 to 7000 ng/ml IGF-BP3, or 3000 to 6000 ng/ml IGF-BP3.

In one embodiment, the human serum product comprises a molar ratio between monomeric (i.e. free or unbound) IGF-1 to IGF-1 bound in macromolecular complexes (of a molecular size of 30 kDa or more) of 1/1000 to 20/1000, or 2/1000 to 15/1000, or 3/1000 to 10/1000. In one embodiment, the human serum product comprises a molar ratio between monomeric (i.e. free or unbound) IGF-1 to IGF-1 bound in macromolecular complexes (of a molecular size of 30 kDa or more) of 20/1000 or less, 15/1000 or less, or 10/1000 or less.

In one embodiment, the human serum product comprises IGFs bound in macromolecular complexes of a molecular size of 30 kDa or more, the product comprising IGF-1 bound to IGF-BP3, at a concentration of 20 to 600 ng/ml IGF-1 and 1300 to 8000 ng/ml IGF-BP3, 2000 to 7000 ng/ml IGF-BP3, or 3000 to 6000 ng/ml IGF-BP3.

In one embodiment, the human serum product comprises IGFs bound in macromolecular complexes of a molecular size of 30 kDa or more, the product comprising IGF-1 bound to IGF-BP3, at a concentration of 20 to 600 ng/ml IGF-1, 30 to 500 ng/ml, 50 to 400 ng/ml, or 100 to 300 ng/ml, and 1300 to 8000 ng/ml IGF-BP3, wherein the concentration of IGF-1 refers to the entire concentration of the IGF-1 protein in the human serum product, i.e. the sum of concentrations of the bound and unbound (free) form.

In one embodiment, the human serum product has a wound-closure performance in an *in vitro* wound-closure assay of 50% or more, normalised to an area of injury, at 35 hours. This means that 50% or more of the initial wound area is closed after 35 hours.

In one embodiment, the human serum product has a wound-closure performance in an *in vitro* wound-closure assay of 50% or more, normalised to an area of injury, at 21 hours. This means that 50% or more of the initial wound area is closed after 21 hours.

The wound-closure performance is based on an *in vitro* wound-closure assay which monitors human corneal epithelial cells (HCE-T cells) over time, wherein the read-out for the reduction (in %) of the wound area is based on HCE-T cells with a well-defined micro-injury (scratch). A detailed description is given in the experimental section of this application.

The *in vitro* wound-closure assay is useful to mimic e.g. dry eye disease and other pathologies, and monitors wound closure with respect to a negative control and a positive control, wherein the negative control is based on serum-free KGM (keratinocyte growth medium-2), and wherein the positive control is based on KGM supplemented with bovine pituitary extract (i.e. 10 pg/ml).

According to the disclosed embodiments, the *in vitro* wound-closure assay comprises the steps (a) providing an *in vitro* cell culture comprising HCE-T cells, (b) employing a micro-injury, e.g. a scratch on the (mono)layer of HCE-T cells, (c) applying the human serum product according to the invention, (d) monitoring the *in vitro* cell culture, e.g. via light microscopy, (e) and quantifying the time-dependent wound closure as wound-closure performance by calculating the percentage difference in the wound area at different time points.

The quantification of the time-dependent wound closure may for example be performed or aided by image analysis software.

In one embodiment, the human serum product has a wound-closure performance in an *in vitro* wound-closure assay of 50% or more, 55% or more, 60% or more, or 65% or more, normalised to an area of injury, at 35 hours. In one embodiment, the human serum product has a wound-closure performance in an *in vitro* wound-closure assay of 90% or less, 85% or less, 80% or less, or 75% or less, normalised to an area of injury, at 35 hours. In one embodiment, the human serum product has a wound-closure performance in an *in vitro* wound-closure assay of 50% to 90%, 55% to 85%, 60% to 80%, or 65% to 75%, normalised to an area of injury, at 35 hours.

In one embodiment, the human serum product has a wound-closure performance in an *in vitro* wound-closure assay of 50% or more, 55% or more, 60% or more, or 65% or more, normalised to an area of injury, at 21 hours. In one embodiment, the human serum product has a wound-closure performance in an *in vitro* wound-closure assay of 90% or less, 85% or less, 80% or less, or 75% or less, normalised to an area of injury, at 21 hours. In one embodiment, the human serum product has a wound-closure performance in an *in vitro* wound-closure assay of 50% to 90%, 55% to 85%, 60% to 80%, or 65% to 75%, normalised to an area of injury, at 21 hours.

In one embodiment, the human serum product has an anti-inflammatory performance in an inflammation assay, wherein the anti-inflammatory performance is normalised to a standardised expression of LPS-induced TNF-α (as a reference base value), wherein the anti-inflammatory performance is 50% or more, and/or wherein the human serum product comprises less than 0.2 mg/dl C-reactive protein (CRP).

The anti-inflammatory performance in an inflammation assay is determined by
- providing macrophages from at least five donors;
- combining the isolated macrophages;
- stimulating the combined macrophages with LPS (lipopolysaccharide) to induce TNF-α secretion which serves as a reference value for inflammation;
- assaying the human serum product in triplicate when stimulating the combined macrophages with LPS which serves as a value for anti-inflammatory performance;
- assaying prednisolone, preferably prednisolone acetate (1.0 wt.%), when stimulating the combined macrophages with LPS which serves as a value for 100% anti-inflammatory performance, i.e. serving as positive control;
- assaying medium when stimulating the combined macrophages with LPS which serves as a value for 0% anti-inflammatory performance, i.e. serving as as negative control; and
- quantifying an anti-inflammatory performance for the human serum product in %, normalised in relation to the positive control and the negative control.

In one embodiment, the human serum product comprises less than 0.2 mg/dl CRP, less than 0.06 mg/dl CRP, or less than 0.02 mg/dl CRP. In one embodiment, the human serum product comprises 0.0002 mg/dl CRP or more, 0.0006 mg/dl CRP or more, or 0.002 mg/dl CRP or more. In one embodiment, the product comprises 0.0002 to 0.2 mg/dl CRP, 0.0006 to 0.06 mg/dl CRP, or 0.002 to 0.02 mg/dl CRP.

CRP can be detected in blood and serum in response to inflammation. Particularly, after inflammation the CRP levels rise in the blood of the human (or mammal). Advantageously, the product comprises low amounts of CRP which avoids and/or mitigates the triggering of an inflammatory response.

The human serum composition may be derived from human serum, and is preferably derived from human allogeneic blood. There are a couple of ways for determining that a serum product is obtained from more than one donor, i.e. it is a multi-donor serum product. In this disclosure, a reference to a "pooled" serum product means that it stems from more than one donor. A multi-donor human serum product is characterised by one or more of the following criteria:
- the presence of antigens indicating that the human serum composition is derived from two or more donors, as classified by the International Society of Blood Transfusion (ISBT);
- the presence of soluble HLA class I molecules indicating that the human serum composition is derived from two or more donors; and
- the presence of glycolipids indicating that the human serum composition is derived from two or more donors.

In one embodiment, the presence of HLA class I molecules indicating that the human serum product is derived from two or more donors is based on a method comprising the steps
- providing a human serum product;
- purifying soluble HLA class I molecules from the human serum composition, such as sHLA-A, sHLA-B, sHLA-C, e.g. by affinity chromatography using polyclonal anti-HLA-A, anti-HLA-B and anti-HLA-C antibodies, to obtain an HLA eluate;
- purifying the HLA eluate, e.g. by gel permeation chromatography, to obtain a purified eluate;
- detecting the soluble HLA class I molecules by Western blotting using anti-HLA-A, anti-HLA-B and anti-HLA-C antibodies;
- performing a tryptic digest on the soluble HLA class I molecules;
- optionally extracting the tryptic-digested soluble HLA class I molecules; and
- analysing the tryptic-digested soluble HLA class I molecules by mass spectrometry (MS).

The skilled person knows methods of protein isolation, purification and extraction. The skilled person also knows that Western blotting requires the previous separation of proteins or peptides according to their size, e.g. by SDS-PAGE. In one embodiment, the extract of tryptic-digested peptides was dried by speed-vacuum and re-suspended in a buffer suitable for MS analysis.

Advantageously, in a multi-donor human serum product, the quantitative amounts of all serum ingredients are levelled and inter-donor variations of the serum ingredients are thus balanced. Potentially harmful serum components, such as donor antibodies (which may trigger a patient reaction), are diluted, thereby minimising any potential risks and/or adverse reactions at the patient's side. Thereby, a product can be obtained or provided which is more standardised than an analogous product based on a human serum composition derived from just one donor. In view of manufacturing the product, using human serum derived from two or more donors also allows scale-up of the method so that larger quantities of the product can be manufactured. Advantageously, working from a multi-donor serum thus allows manufacture and storage of serum-based eye drops as a finished drug.

It is also advantageous that serum obtained from healthy donors can be screened and/or optimised with respect to serum ingredients, for example in relation to tolerability and efficacy, e.g. with respect to establishing well-defined concentration ranges for growth factors.

Several observables allow assessing and discriminating whether a human serum composition is derived from two or more donors, such as the presence of a variety of antigens which indicate the origin from donors with different blood groups. For example, based on antigen analysis, the International Society of Blood Transfusion currently recognises 43 different blood group systems. Furthermore, the presence of glycolipids may indicate that the human serum composition is derived from two or more donors.

In one embodiment, the human serum product comprises one or more growth factors selected from the list of EGF, IGF-BP3, IGF-2, PDGF-BB, Alpha 2-Macroglobulin, KGF, and PEDF. Advantageously, growth factors are capable of stimulating cell proliferation and, in particular, wound healing. Said growth factors may be added to the human serum product as a recombinant protein. Nonetheless, these growth factors may inherently be present in the human serum composition.

In one embodiment, the human serum product comprises one or more, particularly all of the following growth factors in the following concentration ranges:
- 50 to 500 pg/ml EGF, or 20 to 200 pg/ml EGF, optionally quantified according to an EGF-Quantikine Elisa Assay from Bio-Techne Ltd. (Catalog Number DEG00);
- 650 to 4000 ng/ml IGF-BP3, or 260 to 1600 ng/ml IGF-BP3, optionally quantified according to a ligand-binding immune assay;
- 450 to 6000 ng/ml ALS, or 180 to 2400 ng/ml ALS, optionally quantified according to a IGFBP-ALS R&D Elisa (my bioresearch, catalog no. MBS450295);
- 0.37 to 1.49 g/l Alpha 2-Macroglobulin, or 0.15 to 0.6 g/l Alpha 2-Macroglobulin, optionally quantified according to an Elisa IMD Potsdam;
- 471 to 3683 pg/ml PDGF-BB, or 188.4 to 1473.2 pg/ml PDGF-BB, optionally quantified according to an Human Quanitikine Elisa from Bio-Techne Ltd. (Catalog Number DBB00);
- 2 to 100 pg/ml KGF, optionally quantified according to a Quantikine^{®} ELISA from R&D Systems, Inc. (Catalog Number DKG00);
- 1 to 30 ng/ml TGF-β1, or 0.4 to 12 ng/ml TGF-β1, optionally quantified according to an IMD Berlin TGF-beta-1 IVD assay;
- 30 to 222.5 pg/ml VEGF, or 12 to 89 pg/ml VEGF, optionally quantified according to IMD Berlin;
- 60 to 5000 ng/ml PEDF, or 24 to 2000 ng/ml PEDF, optionally quantified according to an ELISA Kit from Bioassay Technology Laboratory R&D; and
- 1750 to 2600 mg/dL albumin, or 700 to 1040 mg/dL albumin, optionally quantified according to the MVZ Labor Volkmann Karlsruhe.

In one embodiment, the human serum product comprises 50 to 500 pg/ml EGF, or 100 to 250 pg/ml EGF, or 20 to 200 pg/ml EGF, or 40 to 150 pg/ml EGF, optionally quantified according to an EGF- Quantikine Elisa Assay from Bio-Techne Ltd. (Catalog Number DEG00).

In one embodiment, the human serum product comprises 50 to 250 ng/ml IGF-1, or 70 to 200 ng/ml IGF-1, or 20 to 100 ng/ml IGF-1, or 30 to 80 ng/ml IGF-1, optionally quantified according to a Diasorin Liaison IGF-1 chemiluminescence assay.

In one embodiment, the human serum product comprises 650 to 4000 ng/ml IGF-BP3, 1000 to 3000 ng/ml IGF-BP3, 260 to 1600 ng/ml IGF-BP3, or 500 to 1200 ng/ml IGF-BP3, optionally quantified according to a ligand-binding immune assay.

In one embodiment, the human serum product comprises 450 to 6000 ng/ml ALS, 1000 to 4000 ng/ml ALS, 180 to 2400 ng/ml ALS, or 500 to 1500 ng/ml ALS, optionally quantified according to a IGFBP-ALS R&D Elisa (my bioresearch, catalog no. MBS450295).

In one embodiment, the human serum product comprises 0.37 to 1.49 g/l Alpha 2-Macroglobulin, 0.55 to 1.20 g/l Alpha 2-Macroglobulin, 0.15 to 0.6 g/l Alpha 2-Macroglobulin, or 0.2 to 0.5 g/l Alpha 2-Macroglobulin, optionally quantified according to an Elisa IMD Potsdam.

In one embodiment, the human serum product comprises 471 to 3683 pg/ml PDGF-BB, 1000 to 3000 pg/ml PDGF-BB, 188.4 to 1473.2 pg/ml PDGF-BB, or 400 to 1200 pg/ml PDGF-BB, optionally quantified according to an Human Quanitikine Elisa from Bio-Techne Ltd. (Catalog Number DBB00).

In one embodiment, the human serum product comprises 2 to 100 pg/ml KGF, 5 to 70 pg/ml KGF, or 10 to 50 pg/ml KGF, optionally quantified according to a Quantikine^{®} ELISA from R&D Systems, Inc. (Catalog Number DKG00).

In one embodiment, the human serum product comprises 1 to 30 ng/ml TGF-β1, 3 to 20 ng/ml TGF-β1, 0.4 to 12 ng/ml TGF-β1, or 1.0 to 8 ng/ml TGF-β1, optionally quantified according to an IMD Berlin TGF-beta-1 IVD assay.

In one embodiment, the human serum product comprises 30 to 222.5 pg/ml VEGF, 50 to 150 pg/ml VEGF, 12 to 89 pg/ml VEGF, or 20 to 60 pg/ml VEGF, optionally quantified according to IMD Berlin.

In one embodiment, the human serum product comprises 60 to 5000 ng/ml PEDF, 300 to 3000 ng/ml PEDF, 24 to 2000 ng/ml PEDF, or 120 to 1200 ng/ml PEDF, optionally quantified according to an ELISA Kit from Bioassay Technology Laboratory R&D.

In one embodiment, the human serum product comprises 1750 to 2600 mg/dl albumin, or 700 to 1040 mg/dl albumin, optionally quantified according to the MVZ Labor Volkmann Karlsruhe.

The skilled person is aware that enzyme-linked immunosorbent assays are the result of conscious design choices and numerous other (sometimes elusive) factors that contribute to the quantitative result. It is therefore imperative to generate a standard curve in order to normalise, i.e. to correct, the read-out values.

Advantageously, albumin prevents precipitation of proteins, such as growth factors, in an aqueous solution and also acts as a radical scavenger having antioxidant properties during storage. Albumin also prevents hydrophobic interactions between individual serum ingredients and towards pharmaceutical container surfaces.

In one embodiment, the multi-donor human serum product comprises insulin-like growth factors (IGFs) bound in macromolecular complexes of a molecular size of 30 kDa or more, the product comprising 20 ng/ml IGF-1 or more, the product comprising less than 70 mg/dl monosaccharides, wherein the product is optionally an isotonic solution or an isotonic gel, wherein the product has a wound-closure performance in an *in vitro* wound-closure assay of 50% or more, normalised to an area of injury, at 35 hours, wherein the product comprises one or more, particularly all of the following growth factors in the following concentration ranges:
- 50 to 500 pg/ml EGF, optionally quantified according to an EGF- Quantikine Elisa Assay from Bio-Techne Ltd. (Catalog Number DEG00);
- 650 to 4000 ng/ml IGF-BP3, optionally quantified according to a ligand-binding immune assay;
- 450 to 6000 ng/ml ALS, optionally quantified according to a IGFBP-ALS R&D Elisa (my bioresearch, catalog no. MBS450295);
- 0.37 to 1.49 g/l Alpha 2-Macroglobulin, optionally quantified according to an Elisa IMD Potsdam;
- 471 to 3683 pg/ml PDGF-BB, optionally quantified according to an Human Quanitikine Elisa from Bio-Techne Ltd. (Catalog Number DBB00);
- 2 to 100 pg/ml KGF, optionally quantified according to a Quantikine^{®} ELISA from R&D Systems, Inc. (Catalog Number DKG00);
- 1 to 30 ng/ml TGF-β1, optionally quantified according to an IMD Berlin TGF-beta-1 IVD assay;
- 30 to 222.5 pg/ml VEGF, optionally quantified according to IMD Berlin;
- 60 to 5000 ng/ml PEDF, optionally quantified according to an ELISA Kit from Bioassay Technology Laboratory R&D; and
- 1750 to 2600 mg/dl albumin, optionally quantified according to the MVZ Labor Volkmann Karlsruhe.

In one embodiment, the multi-donor human serum product comprises insulin-like growth factors (IGFs) bound in macromolecular complexes of a molecular size of 30 kDa or more, the product comprising 20 ng/ml IGF-1 or more, the product comprising less than 70 mg/dl monosaccharides, wherein the product is optionally an isotonic solution or an isotonic gel, wherein the product has a wound-closure performance in an *in vitro* wound-closure assay of 50% or more, normalised to an area of injury, at 35 hours, wherein the product comprises one or more, particularly all of the following growth factors in the following concentration ranges:
- 20 to 200 pg/ml EGF, optionally quantified according to an EGF- Quantikine Elisa Assay from Bio-Techne Ltd. (Catalog Number DEG00);
- 260 to 1600 ng/ml IGF-BP3, optionally quantified according to a ligand-binding immune assay;
- 180 to 2400 ng/ml ALS, optionally quantified according to a IGFBP-ALS R&D Elisa (my bioresearch, catalog no. MBS450295);
- 0.15 to 0.6 g/l Alpha 2-Macroglobulin, optionally quantified according to an Elisa IMD Potsdam;
- 188.4 to 1473.2 pg/ml PDGF-BB, optionally quantified according to an Human Quanitikine Elisa from Bio-Techne Ltd. (Catalog Number DBB00);
- 2 to 100 pg/ml KGF, optionally quantified according to a Quantikine^{®} ELISA from R&D Systems, Inc. (Catalog Number DKG00);
- 0.4 to 12 ng/ml TGF-β1, optionally quantified according to an IMD Berlin TGF-beta-1 IVD assay;
- 12 to 89 pg/ml VEGF, optionally quantified according to IMD Berlin;
- 24 to 2000 ng/ml PEDF, optionally quantified according to an ELISA Kit from Bioassay Technology Laboratory R&D; and
- 700 to 1040 mg/dl albumin, optionally quantified according to the MVZ Labor Volkmann Karlsruhe.

In one embodiment, the human serum product comprises insulin-like growth factors (IGFs) bound in macromolecular complexes of a molecular size of 30 kDa or more, the product comprising 20 to 100 ng/ml IGF-1, the product comprising less than 70 mg/dl monosaccharides, wherein the product is optionally an isotonic solution or an isotonic gel, wherein the product has a wound-closure performance in an *in vitro* wound-closure assay of 50% or more, normalised to an area of injury, at 35 hours, wherein the product comprises one or more, particularly all of the following growth factors in the following concentration ranges:

### Medical use

In a second aspect, the invention relates to a product for use in medicine.

Due to its composition, the human serum product according to the invention stimulates cell proliferation and exerts epithelial defects healing properties. The human serum product can thus be employed for medical and pharmaceutical purposes, for example after inflammation and/or injury. Similarly, the human serum product may be employed after surgery to assist and/or to accelerate recovery.

In a related aspect, the invention relates to the human serum product for use in the treatment of dry eye disease.

In one embodiment, the human serum product according to this disclosure is for use in the treatment of macular degeneration, epithelial cornea damage, conditions resulting from or after cornea transplantation, conditions relating to or resulting from wound healing disorders, or conditions relating to or resulting from ophthalmic laser treatment.

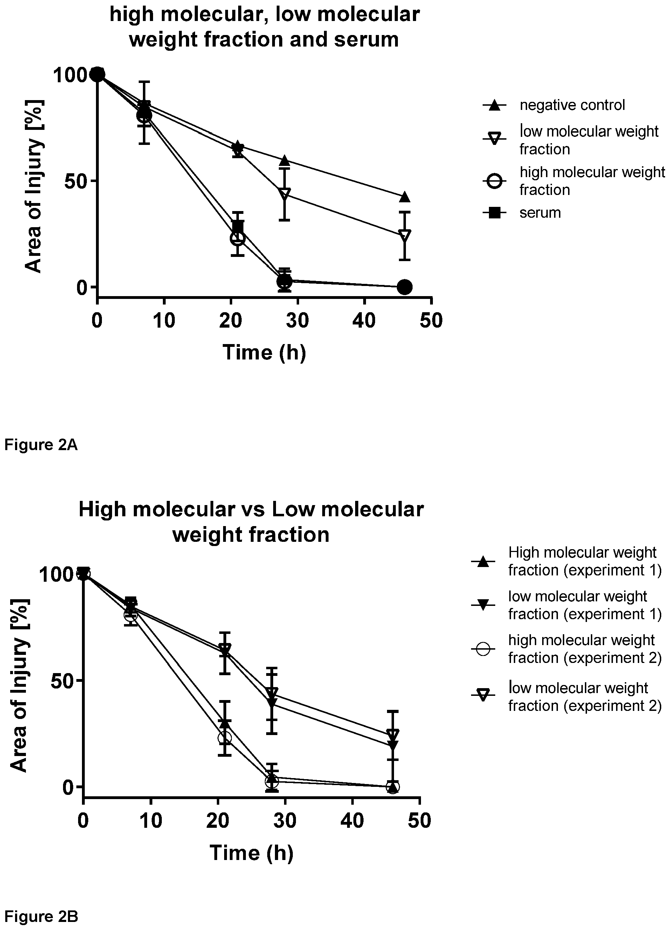

The inventors have shown that the human serum product outperforms current compositions and provides for an improved epithelial defect healing performance (Figures 2A to 4). The described assay has been shown to be a useful model for dry eye disease and related pathologies (Hahne M, Reichl S, Simulation of corneal epithelial injuries by mechanical and corrosive damage: Influence of fetal bovine serum and dexpanthenol on epithelial regeneration in a cell culture model, Ophthalmologe 107, 2010, 529-532, and 534-536). The product hence provides for an improved therapy against dry eye disease and related pathologies.

In one embodiment of the human serum product for use in medicine, or the human serum product for use in the treatment of dry eye disease, the human serum product is derived from allogeneic blood.

In one embodiment of the human serum product for use in the treatment of macular degeneration, epithelial cornea damage, conditions resulting from or after cornea transplantation, conditions relating to or resulting from wound healing disorders, or conditions relating to or resulting from ophthalmic laser treatment, the human serum product is derived from allogeneic blood.

Allogeneic blood may be obtained by providing and/or pooling blood from a multitude of donors. Such allogeneic blood donations are advantageous for the manufacture of medication, i.e. pharmaceutical products, because they balance the inter-donor composition variations of the blood and allow scaling-up of the manufacture. Further, blood from a multitude of donors can be selected so as to obtain the desired concentrations as set out herein. For example, blood and/or serum of single donors can be examined for IGF contents and used for pooling, if a minimum IGF level is present.

In one aspect, the invention relates to an ophthalmic composition, such as eye drops or gels, comprising the product. Eye drops and gels represent a typical formulation that is useful for the treatment of e.g. dry eye disease or related pathologies or similar pathologies.

### Method for manufacturing a product

In one aspect, the invention relates to a method for manufacturing a human serum product, comprising the steps of
- providing human allogeneic blood volume portions from a multitude of donors,
- selecting volume portions from at least two donors,
- deriving serum from the selected volume portions to obtain serum portions,
- pooling the selected serum portions to obtain pooled serum, wherein optionally the selected serum portions are from donors all having an age of 45 years or less;
- obtaining an intermediate product from the pooled serum, the intermediate product comprising 100 ng/ml IGF-1 or more; and
- obtaining the human serum product according to this disclosure from the intermediate product.

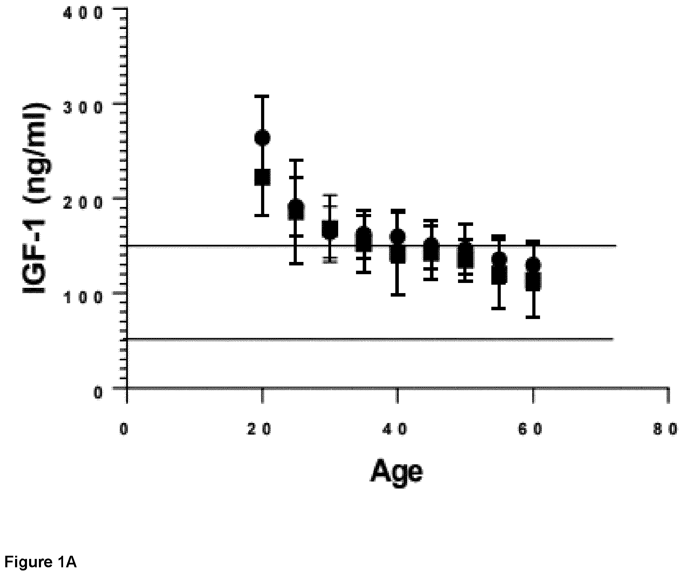

The method relies on providing whole blood from a multitude of human donors which is selected and pooled in such a way to ensure a sufficient IGF concentration, such that the intermediate product comprises 100 ng/ml IGF-1 or more. It was established that the IGF-1 concentration in human allogeneic blood varies significantly and decreases with an increasing lifetime of the donors (Figure 1A). Hence, restricting the donor age of the multitude of donors to 45 years or less contributes in ensuring an efficient process as less volume portions have to be discarded. The selecting step ensures a sufficient IGF-1 concentration in the human serum product.

In one embodiment of the method, the intermediate product is serum, wherein obtaining the intermediate product comprises preparing serum from the human allogeneic blood.

In one embodiment of the method, the intermediate product is a retentate of an ultrafiltration process, wherein obtaining the intermediate product comprises preparing serum from the human allogeneic blood, ultrafiltrating the serum to obtain a filtrate and a retentate, and selecting the retentate.

In one embodiment of the method, obtaining the human serum product comprises diluting the intermediate product.

In one embodiment, dilution of the intermediate product comprises dilution of the intermediate product, e.g. serum (from the human allogeneic blood), to 10 to 30 vol.%, or 15 to 25 vol.%, or about 20 vol.%, with an essentially isotonic solution, e.g. a NaCl solution.

In one embodiment, the product may be obtained by addition of recombinant proteins, such as e.g. recombinant growth factors.

In one embodiment of the method, the ultrafiltration process is carried out with a molecular cut-off membrane of at least 20 kDa, or at least 30 kDa, or at least 50 kDa, or at least 100 kDa. It was found that the retentate comprises insulin-like growth factors (IGFs) bound in macromolecular complexes of a molecular size of 30 kDa or more. Advantageously, the method provides for obtaining a product which has improved therapeutic properties, e.g. for wound healing, and anti-inflammation properties.

In one embodiment of the method, the obtained product has an anti-inflammatory performance in an inflammation assay, wherein the anti-inflammatory performance is normalised to a standardised expression of LPS-induced TNF-α (as a reference base value), wherein the anti-inflammatory performance is 50% or more, and/or wherein the product comprises less than 0.2 mg/dl C-reactive protein (CRP).

In one embodiment, obtaining serum from the human allogeneic blood comprises allowing coagulation, also referred to as clotting, and removing the clot and any cells present in the blood. Optionally, removing the clot and any cells present in the blood is performed by centrifugation. The resulting yellowish liquid supernatant is the serum.

In an alternative embodiment, obtaining serum from the human allogeneic blood comprises providing human allogeneic blood volume portions from a multitude of donors, selecting volume portions from at least two donors, obtaining serum portions from the selected volume portions, and pooling the selected serum portions, wherein optionally the selected volume portions are from donors all having an age of 45 years or less. In this alternative embodiment, not blood but serum is pooled. Other than this modification, the remaining method steps may be the same as in the method according to the other embodiments of this disclosure.

In one embodiment, obtaining the product comprises adding a solution to the intermediate product, wherein the solution optionally comprises ingredients and or additives, such as e.g. vitamins, growth factors, pH modifiers, buffering agents, surfactants, viscosity modifiers, and/or wherein the solution comprises NaCl in an amount so that the product is isotonic with respect to its intended pharmaceutical application.

The methods according to this disclosure are advantageous because the obtained products do not require the addition of stabilisers as part of the manufacturing. Whereas typical pharmaceutical products may require preserving agents, such as e.g. benzalkonium chloride (which is an irritant to the eyes), the methods according to this disclosure work in the absence of such agents. The intermediate product obtained from the human allogeneic blood is, thanks to its way of manufacture, not prone to microbial contamination, and can thus be manufactured, further processed and stored in the absence of preserving agents.

In one embodiment, a method for manufacturing a product is provided, comprising the steps of
- providing human allogeneic blood volume portions from a multitude of donors,
- selecting volume portions from at least two donors,
- deriving serum from the selected volume portions to obtain serum portions,
- pooling the selected serum portions to obtain pooled serum, wherein optionally the selected serum portions are from donors all having an age of 45 years or less;
- obtaining an intermediate product from the pooled serum, the intermediate product comprising 100 ng/ml IGF-1 or more; and
- obtaining the human serum product according to this disclosure in the form of an alginate dressing from the intermediate product.

Advantageously, an alginate dressing allows the slow controlled release of active serum ingredients and/or recombinant growth factors that may be added to the alginate dressing. An alginate dressing serves as a natural wound dressing and contains carbohydrate sources that cannot be metabolised by human. An alginate is a form of an isotonic gel, wherein the alginate dressing is soft and has thixotropic properties. Cells and/or growth factors can be embedded and/or incorporated, respectively, into said isotonic gel. To maintain a constant dosing profile in the alginate, the use of ocular inserts provides a feasible method to deliver the active ingredients in a controlled manner to the eye over a period of several hours. The insert is placed in the lower eye portion. Sodium alginate is preferably chosen as a matrix material due to its solubility in water and ease for being cross-linked at room temperature through the addition of Ca²⁺ ions.

In one embodiment of the method, the multitude of donors all have an age of 45 years or less, 40 years or less, preferably 30 years or less. In one embodiment of the method, the multitude of donors all have an age of 18 years or more, or 20 years or more. In one embodiment of the method, the multitude of donors all have an age of 18 to 45 years, 18 to 40 years, or 20 to 30 years. It is advantageous to select for donors which, by virtue of their age, have a sufficiently high IGF-1 concentration in their human serum (cf. Figure 1A).

In one embodiment of the method, the intermediate product comprises less than 140 mg/dl monosaccharides, and/or 10 to 200 mg/dl total cholesterol, wherein total cholesterol is the sum of LDL (low-density lipoprotein), HDL (high-density lipoprotein) and VLDL (very low-density lipoprotein).

In one embodiment, the method comprises a virus removal step, for example by means of virus filtration and/or inactivation.

### Intermediate product

In an aspect related to the human serum product not according to the claimed invention, this disclosure provides an intermediate product comprising insulin-like growth factors (IGFs) bound in macromolecular complexes of a molecular size of 30 kDa or more, wherein the intermediate product comprises 100 to 500 ng/ml IGF-1.

In one embodiment not according to the claimed invention, the intermediate product comprises IGF-1 at a concentration of 100 ng/ml or more, 150 ng/ml or more, 250 ng/ml or more, or 500 ng/ml or more. In one embodiment, the intermediate product comprises IGF-1 at a concentration of 3000 ng/ml or less, 2500 ng/ml or less, 2000 ng/ml or less, or 1500 ng/ml or less. In one embodiment, the intermediate product comprises IGF-1 at a concentration of 100 to 3000 ng/ml, 150 to 2500 ng/ml, 250 to 2000 ng/ml, or 500 to 1500 ng/ml.

In one embodiment not according to the claimed invention, the intermediate product comprises 6500 ng/ml IGF-BP3 or more, 10000 ng/ml IGF-BP3 or more, or 15000 ng/ml IGF-BP3 or more. In one embodiment, the intermediate product comprises 40000 ng/ml IGF-BP3 or less, 35000 ng/ml IGF-BP3 or less, or 30000 ng/ml IGF-BP3 or less. In one embodiment, the intermediate product comprises 6500 to 40000 ng/ml IGF-BP3, 10000 to 35000 ng/ml IGF-BP3, or 15000 to 30000 ng/ml IGF-BP3.

In one embodiment not according to the claimed invention, the intermediate product comprises IGFs bound in macromolecular complexes of a molecular size of 30 kDa or more, the product comprising IGF-1 bound to IGF-BP3, at a concentration of 100 to 3000 ng/ml IGF-1 and 6500 to 40000 ng/ml IGF-BP3, 10000 to 35000 ng/ml IGF-BP3, or 15000 to 30000 ng/ml IGF-BP3.

In one embodiment not according to the claimed invention, the intermediate product comprises IGFs bound in macromolecular complexes of a molecular size of 30 kDa or more, the product comprising IGF-1 bound to IGF-BP3, at a concentration of 100 to 3000 ng/ml, 150 to 2500 ng/ml, 250 to 2000 ng/ml, or 500 to 1500 ng/ml IGF-1, and 6500 to 40000 ng/ml IGF-BP3.

### Detailed description of the drawings

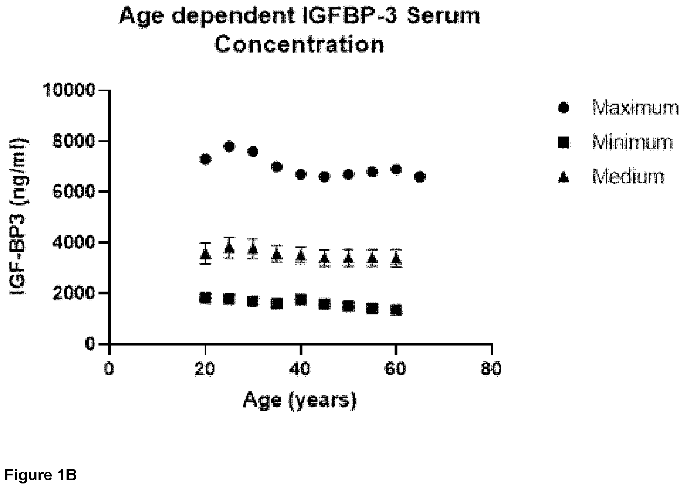

**Figure 1A** and **Figure 1B** show, respectively, the age-dependant IGF-1 concentration and IGF-BP3 concentration in human serum, evidencing that the IGF-1 concentration in human serum decreases with increasing lifetime of the donors, while IGF-BP3 decreases only mildly over age. The age is given in years. The round circles indicate clinical diagnostic data, and the squares indicate published data (Growth Hormone & IGF Research 18 (2008): 228-237). The data plead for a blood donor age restriction to below 45 years. A serum pool adjusted to a concentration of more than 130 ng/ml IGF-1 to 280 ng/ml IGF-1 was stable for up to 24 months monitored by the concentration of active IGF-1 (+/- 20%).

**Figure 2** shows the activity of serum fractions in an *in vitro* wound closure assay. The *in vitro* wound closure assay monitors human corneal epithelial cells (HCE-T cells) over time, wherein the read-out for the reduction (in %) of the wound area is based on HCE-T cells with a well-defined micro-injury (scratch). Ultrafiltration of pooled serum yielded a separation of high-molecular weight contents (in a retentate) from low-molecular weight contents (in a filtrate). The data indicate that, off the serum, the high-molecular weight fraction provides for better, i.e. faster, wound healing than the low-molecular weight fraction, in comparison to unfiltered serum and a negative control **(****Figure 2A****).** Two independent experiments 1 and 2 confirmed the different activity between the high-molecular weight fraction and the low-molecular weight fraction **(****Figure 2B****).**

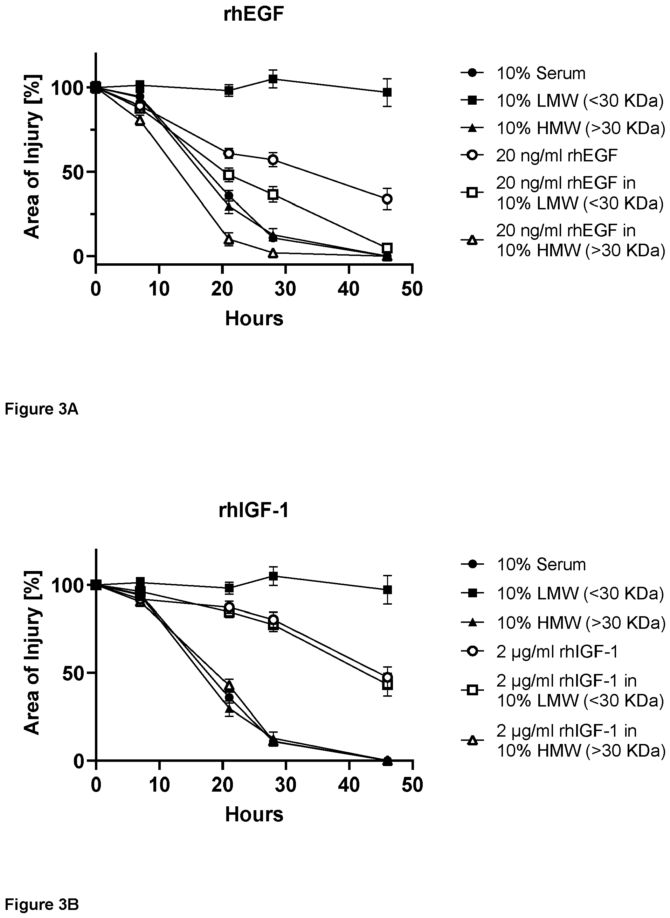

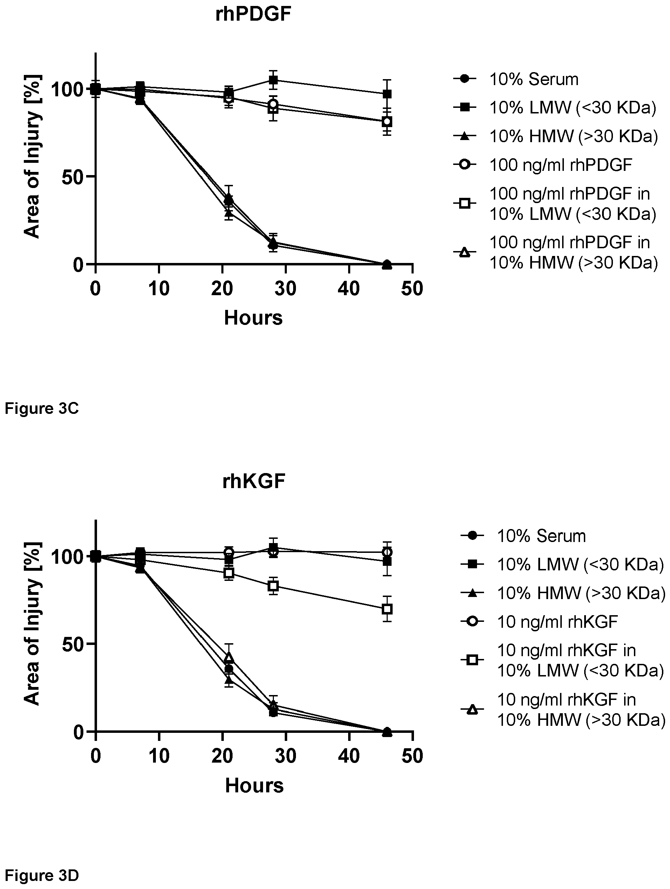

**Figure 3** shows the effect of five different (recombinant) human (rh) growth factors, i.e. EGF **(****Figure 3A****),** IGF-1 **(****Figure 3B****),** PDGF **(****Figure 3C****),** and KGF **(****Figure 3D****),** on wound closure when combined with the high-molecular weight (HMW) fraction or the low-molecular weight fraction (LMW) of ultra-filtered pooled human serum, respectively. The effect was determined as wound closure over the next 50 hours. Results indicate that the potency to induce corneal epithelial cell migration and proliferation is similar in serum and HMW fraction-90 % wound closure is observed at time point 28 hrs, while no effect on wound closure is observed for LMW fraction. These results indicate that biological active ingredients are predominantly present in the HMW fraction.

Single rh-growth factors are listed in the order of their increasing effect on wound closure-PDGF; KGF; IGF-1 and EGF. Wound closure is further accelerated, when rh- growth factors are combined with HMW and surprisingly also with LMW, and is highest for EGF. These results indicate that HMW as well as LMW ingredients act synergistically with all rh- growth factors analysed, besides PDGF.

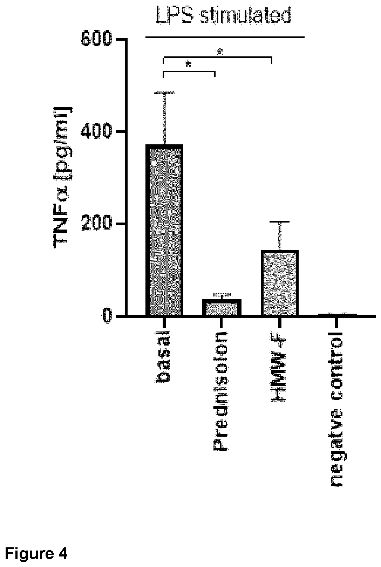

**Figure 4** shows the anti-inflammatory effect of three high-molecular weight fraction samples, after storage at -20 °C for 6 years, in the Lipopolysaccharide (LPS)-TNF-alfa inhibition assay in comparison to prednisolone (positive control). Monocytes are isolated of heparin blood and stimulated with lipopolysaccharide. Lipopolysaccharide is a surface molecule of Gram-negative bacteria, which induces a significant inflammatory response, and TNF-alfa secretion by binding to their CD14 molecules. The standardized expression of LPS-induced TNF-alfa (as a base value) is used as a reference value. In replicate controls, the LPS-induced TNF-alfa secretion is analysed under the influence of added preparations. The read-out below the base value indicates an anti-inflammatory effect. Values above the base value indicate a pro-inflammatory *in vitro* effect of the respective preparation. Prednisolon acetate (1.0 wt.%) (glucocorticoid) was used as positive control.

### Methods

### Allogeneic blood and serum

Venous blood was collected from healthy volunteers via venepuncture after written informed consent and approval by the Ethics Committee, with up to 500 ml per donation, into blood bags free of anti-coagulant. Serum was prepared by keeping the collected whole blood at room temperature for at least 2 hours to allow complete clotting, as confirmed in a PTT (partial thromboplastin time) assay followed by storage at 4 to 8 °C for up to 24 hours. Clotted blood was centrifuged twice at 3000 g for 10 min at room temperature. The resulting supernatant (serum) was separated from the blood cake using a manual extractor, pooled and diluted to a final concentration of 20 vol.% in an isotonic NaCl solution. Serum was dispensed in 5 or 10 ml aliquots following pathogen inactivation via virus filtration and sterile filtration in a 3K-multidose applicator.

### Ultrafiltration of pooled serum

Pooled serum (1 L) was diluted with NaCL (0.9%) and ultrafiltrated using 30 kDa and 100 kDa polyethersulfone (PES) membranes (PALL Centramate T-series, Dreieich, Germany) using a flow rate of 100 ml/min by a multiple use pump from Almatechnik (Zeiningen, CH). The ultrafiltration was stopped, when the retentate volume corresponded to 10 % of the starting volume.

### Wound-closure assay

The stimulation of corneal epithelial injury and its regeneration was assayed using an in vitro model of immortalized human corneal epithelial cells (HCE-T) grown as monolayer cell cultures. Briefly, 4×10⁴ cells were seeded in 24 well-plates and cultured until the cells formed an optically confluent monolayer at 37 °C in a humidified atmosphere containing 5% CO₂ using a serum-free growth medium (KGM). Frozen serum samples, KGM (keratinocyte growth medium-2, Lonza), supplements and PBS (phosphate-buffered saline) were brought to room temperature. The epithelial cell model was then damaged by mechanical injury using a sterile 200 µl plastic pipette tip to scratch a wound through the centre of the well to create a 800 to 900 µm cell-free wound and washed twice with PBS (1 ml per well) to remove floating cells. The size of the damage was confirmed by light-optical microscopy (Feng Y et al., Epithelial Wound Healing on Keratin Film, Amniotic Membrane and Polystyrene In Vitro, Current Eye Research 2014; 39(6):561-570). Subsequently the epithelial cell model was further cultivated using serum-free KGM supplemented with different ingredients, i.e. serum, high-molecular weight fraction, low-molecular weight fraction, and recombinant human growth factors. Wound healing was evaluated using a photomicroscope. Six photos per time point were taken over a period of up to 60 hours. The regeneration enhancing effect was calculated as the percentage difference in the wound width at different time points. HCE- T cells cultured in KGM served as a negative control. HCE- T cells cultured in KGM supplemented with bovine pituitary extract (10 µg/ml) served as a positive control.

### Inflammation assay

Anti-inflammatory properties of serum preparations were assayed by a TNF-α inhibition test. Tumour necrosis factor alpha (TNF-α) is a cytokine involved in almost any inflammation reaction. Macrophages were isolated form at least five donors. The combined macrophages were stimulated with LPS (lipopolysaccharide) which is a surface molecule of gram-negative bacteria which induces a significant inflammatory response, i.e. TNF-α secretion in the macrophages by binding to their CD14 receptor molecules. As a reference value for inflammation, this standardised LPS-induced TNF-α release was used. The products according to the invention were assayed (n = 3) in comparison with respect to the LPS-induced TNF-α secretion. TNF-α secretion concentration values below the reference value were considered to have an anti-inflammatory effect; values larger than that were considered to have a pro-inflammatory effect. Prednisolone acetate (1.0 wt.%) was used as positive control, and medium was used as negative control, respectively, indicating 100% and 0% inflammation reduction.

### HLA-class I assay

Tryptic digest of HLA-class I molecules provides an approximately 90% sequence coverage and to thus distinguish between HLA class I molecules from different serum donors. HLA-class I molecules are highly polymorphic molecules, which greatly differ in human individuals within a population. Therefore, the described method for the detection of soluble HLA-class I (sHLA) molecules in serum was developed. The entire HLA gen complex comprises approximately 4.000 kilobases and is generally divided into two genetic regions, of which the class I genes encompass *sHLA-A, sHLA-B,* and *sHLA-C.* sHLA-A, sHLA-B, sHLA-C serum molecules were purified from 10 ml serum by affinity chromatography using polyclonal anti-HLA-A, anti-HLA-B and anti-HLA-C antibodies. Subsequently the eluates were further purified by gel permeation chromatography, and the pooled fractions detected by SDS-PAGE and subsequent Western blotting using anti-HLA-A, anti-HLA-B and anti-HLA-C antibodies. The PVDF blot was blocked into 0.5% (w/v) PVP-40 at room temperature for 30 min. After cutting the PVDF membranes with the HLA-A, HLA-B and HLA-C protein spots, the PVDF membranes were wetted by methanol, washed 10 times with water. Subsequently the blots were reduced and alkylated with, respectively, 10 mM DTT and 55 mM IDA, sequentially. Then the blots were immersed into 25 µl 25 mM NH₄HCO₃, heated at 95 °C for 5 minutes to denature the proteins. Subsequently, 25 µl of trypsin solution (10 µl of 40 ng/µl trypsin, 5 µl of 5% Tween-20 and 10 µl of acetonitrile) was added and incubated at 37 °C overnight. The trypsin-digested peptides were extracted from the blot three times with 40 µl of 5% (v/v) trifluoroacetic acid and 45 % H₂0 50% (v/v) ACN at room temperature for 1 hour. The extract was dried by speed-vacuum and re-suspended in 20 µl of 0.1% TFA for mass spectrometry analysis.

Mass spectrometric sequence analysis of HLA-class I molecules was based on detection of tryptic peptides. Samples were measured using an LTQ Orbitrap Velos Pro system (Thermo Fisher Scientific, Bremen, Germany) online-coupled to a U3000 RSLCnano (Thermo Fisher Scientific, Idstein, Germany) employing an Acclaim PepMap analytical column (75 µm x 500 mm, 2 µm, 100 A, Thermo Fisher Scientific, Bremen, Germany) at a flow rate of 250 nl/min. MS/MS measurement was done after carbamidomethylation and digestion with trypsin for sequencing of the proteolytic fragments. Separation was carried out using a linear acetonitrile gradient. The software 2.2 SP1.48 (Thermo Fisher Scientific, Bremen, Germany) was used for data-dependent tandem mass spectrometry (MS/MS) analyses (https://www.sciencedirect.com/topics/biochemistry-genetics-and-molecular-biologv/sequest). Extracted ion chromatograms (XICs) for the standard peptides and the analyte peptides were generated using Qual Browser embedded in the XCalibur software suite. For peptide identification, MS/MS spectra were correlated with the UniProt human reference proteome set (http://www.uniprot.org). Theoretical average molecular masses were calculated with Prot-Param (https://web.expasy.org/protparam/).

### Examples

### Production of multi-donor human serum eye drops

Briefly, up to 500 ml of venous whole blood was collected from male donors (n=5) in the age from 18 to 40 years. Blood was collected into multi-bag blood collection bags containing no anti-coagulant (Compoflex Fresenius). Blood was allowed to clot during storage at 18-22 °C (RT) for at least 2 hours followed by storage at 2-8 °C. Within 24 h following blood collection, serum was separated from the blood cake by centrifugation at 3000 x g for 10 min. at RT, twice. The resulting supernatant (serum) was extracted using a manual extractor. Serum was pooled (1L) to obtain the intermediate product and samples were taken for Quality Control Analysis. The intermediate serumpool was adjusted to the corresponding concentration [(50 % or 20 % by diluting with NaCl (0,9%)] and dispensed in 5 ml aliquots by using a graduated syringe with filter (0,22µm) in Aero Pump ophthalmic multidose 3K^{®}-Systems under clean room conditions class A/B, followed by release controls. Subsequently, the eye drop bottles were frozen and stored at temperatures at or below -20 °C until use.

### Production of an intermediate product by ultrafiltration obtaining a high - and a low molecular fraction

Pooled intermediate serum product (1L), was obtained as described in example 1). The serumpool was subsequently separated in a low (LMWF) and high molecular weight fraction (HMWF) by ultrafiltration. Therefore, pooled serum was diluted with 0,9% NaCl and ultrafiltrated using polyethersulfone (PES) membranes with cutoffs of 30 kDa and 100 kDa, respectively (PALL Centramate T-series, Dreieich, Germany) with a flow rate of 100 ml/min by using a multiple use pump from Almatechnik (Zeiningen, CH). The ultrafiltration was stopped, when the retentate volume corresponded to 10 % of the starting volume. Samples were taken from serum pool and the corresponding HMWF and LMWF and analyzed in more detail by testing aliquots on regenerative epithelial potency as well as anti-inflammatory potency.

### Comparison of the regenerative epithelial potency of unfiltered multi-donor serum pool with low- (LMWF) and high-molecular weight fractions (HMWF) in the corneal HCE-T cell assay

**Figure 2** shows the activity of serum fractions in an *in vitro* wound closure assay. The *in vitro* wound closure assay monitors human corneal epithelial cells (HCE-T cells) over time, wherein the read-out for the reduction (in %) of the wound area is based on HCE-T cells with a well-defined micro-injury (scratch). Ultrafiltration of pooled serum yielded a separation of high-molecular weight contents (in a retentate) from low-molecular weight contents (in a filtrate). The data indicate that, off the serum, the high-molecular weight fraction provides for better, i.e. faster, wound healing than the low-molecular weight fraction, in comparison to unfiltered serum and a negative control **(****Figure 2A****).** Two independent experiments 1 and 2 confirmed the different activity between the high-molecular weight fraction and the low-molecular weight fraction **(****Figure 2B****).**

### Detection of IGF-1 bound in macromolecular complexes of multi-donor human serum product in the HMWF fraction (retentate)

HMWF samples were measured using an LTQ Orbitrap Velos Pro system (see Methods, section 'HLA-class I assay'). This approach led to the identification of albumin, IGF-1 BP3 ALS complex, alpha-2 Macroglobulin, PDGF-BB, TGF-beta 1, VEGF and PEDF. The corresponding filtrates (30 kDa and 100 kDa) were analyzed for the concentration of selected physiological ingredients as shown in the table below.

### Characterization of intermediate products

In a first approach, the concentration of the mentioned ingredients was determined experimentally under laboratory conditions. Next, the concentration of selected ingredients was determined under contract in an accredited diagnostic laboratory (MVZ Karlsruhe). Results are presented in Table 1. The regenerative capacity to close epithelial defects was analyzed in an *in vitro* wound-healing assay (HCE-T cell assay). The anti-inflammatory capacity was determined in an TNF-alfa inhibition test.

**Table 1: Concentrations determined in mulitdonor intermediate products**

| **Ingredient** | **Multidonor Serumpool** | **30 kDa Low molecular weight fraction** | **100 kDa Low molecular weight fraction** | **Assay** |
|---|---|---|---|---|
| IGF-1 | 181.5 ng/ml | ≤ 0,5 µg/ ml | ≤ 0,5 µg/ ml | DiasorinLiaison IGF-1 Chemiluminiszenz Assay |
| IGF.BP3 | 5,2 µg/ ml | ≤ 0,5 µg/ ml | ≤ 0,5 µg/ ml | Immulite 2000; Siemens |
| Albumin | 4600 mg/dl | n.d. | n.d. | Photometry |
| Cholesterol | 203.1 mg/dl | n.a. | n.d. | Photometry |
| LDL- Cholesterol | 114.9 mg/dl | n.d. | n.d. | Photometry |
| HDL - Cholesterol | 54.0 mg/dl | n.d. | n.d. | Photometry |
| CRP- C reactive protein | 0.92 mg/l | 0.04 mg/l | n.d. | Turbidimetry (ultrasensitive assay) |
| Glucose | 20 mg/dl | n.a. | n.a. | Photometry |
| Sterility Ph.Eur. 2.6.1 | No growth | - | - | Ph.Eur. method |

| | | | | |
|---|---|---|---|---|
| n.d. = below test detection limit of assay | | | | |

### Product formulation and characterization

The intermediate (serum pool) was adjusted to the required concentration using NaCl (0.9 %). The resulting ingredient content and additional QC analysis, performed to characterize the product are presented in Table 2.

**Table 2**

| **Ingredient** | **Serum (50%) in 0.9% NaCl (V/V)** | **Serum (20%) in 0.9% NaCl (V/V)** | **Assay** |
|---|---|---|---|
| IGF-1 | 90.8 ng/ml | 36.3 ng/ ml | DiasorinLiaison IGF-1 Chemiluminiszenz Assay |
| IGF.BP3 | 2.6 µg/ ml | 1.0 µg/ml | Immulite 2000; Siemens |
| Albumin | 2300 mg /dl | 900 mg /dl | Photometry |
| Cholesterol | 101.6 mg/dl | 40.6 mg/dl | Photometry |
| LDL- Cholesterol | 57.4 mg/dl | 22.9 mg/dl | Photometry |
| HDL - Cholesterol | 27.0 mg/dl | 10.8 mg/dl | Photometry |
| CRP- C reactive protein | 0.45 mg/l | 0.2 mg/l | Turbidimetry (ultrasensitive assay) |
| Glucose | 10.0 mg/dl | 4 mg/dl | Photometry |
| aPPt [sec.] activated partial thromboplastin time | - | | Coagulation assay |
| Residual cells (WBC, RBC, MN, PMN) | 0 | | Differential blood count analyzer |
| Integrity testing of membrane filter | Passed | | Ph.Eur. method |
| Sterility Ph.Eur. 2.6.1 | No growth | | Ph.Eur. method |

## Claims

1. Multi-donor human serum product comprising insulin-like growth factors (IGFs) bound in macromolecular complexes of a molecular size of 30 kDa or more, the product comprising 20 ng/ml IGF-1 or more,
the product comprising less than 70 mg/dl monosaccharides,
wherein the product is optionally an isotonic solution or an isotonic gel.

2. Product according to claim 1, wherein the product comprises less than 40 mg/dl total cholesterol, wherein total cholesterol is the sum of LDL (low-density lipoprotein) and HDL (high-density lipoprotein).

3. Product according to claim 1 or claim 2, wherein the product has a wound-closure performance in an *in vitro* wound-closure assay of 50% or more, normalised to an area of injury, at 35 hours, wherein the *in vitro* wound-closure assay comprises the steps
(a) providing an in vitro cell culture comprising HCE-T cells,
(b) employing a micro-injury, e.g. a scratch on the (mono)layer of HCE-T cells,
(c) applying the human serum product,
(d) monitoring the in vitro cell culture, e.g. via light microscopy, and
(e) quantifying the time-dependent wound closure as wound-closure performance by calculating the percentage difference in the wound area at different time points.

4. Product according to any one of the preceding claims, wherein the product has an anti-inflammatory performance in an inflammation assay, wherein the anti-inflammatory performance is normalised to a standardised expression of LPS-induced TNF-α (as a reference base value), wherein the anti-inflammatory performance is 50% or more,
wherein the anti-inflammatory performance in an inflammation assay is determined by
- providing macrophages from at least five donors;
- combining the isolated macrophages;
- stimulating the combined macrophages with LPS (lipopolysaccharide) to induce TNF-α secretion which serves as a reference value for inflammation;
- assaying the human serum product in triplicate when stimulating the combined macrophages with LPS which serves as a value for anti-inflammatory performance;
- assaying prednisolone, preferably prednisolone acetate (1.0 wt.%), when stimulating the combined macrophages with LPS which serves as a value for 100% anti-inflammatory performance, i.e., serving as positive control;
- assaying medium when stimulating the combined macrophages with LPS which serves as a value for 0% anti-inflammatory performance, i.e., serving as a negative control; and
- quantifying an anti-inflammatory performance for the human serum product in %, normalised in relation to the positive control and the negative control,
and/or wherein the product comprises less than 0.2 mg/dl C-reactive protein (CRP).

5. Product according to any one of the preceding claims, comprising one or more growth factors selected from the list of EGF, IGF-BP3, IGF-2, PDGF-BB, Alpha 2-Macroglobulin, KGF, and PEDF.

6. Product according to any one of the preceding claims, comprising one or more growth factors in the following concentration ranges:
- 50 to 500 pg/ml EGF, or 20 to 200 pg/ml EGF;
- 650 to 4000 ng/ml IGF-BP3, or 260 to 1600 ng/ml IGF-BP3;
- 450 to 6000 ng/ml ALS, or 180 to 2400 ng/ml ALS;
- 0.37 to 1.49 g/l Alpha 2-Macroglobulin, or 0.15 to 0.6 g/l Alpha 2-Macroglobulin;
- 471 to 3683 pg/ml PDGF-BB, or 188.4 to 1473.2 pg/ml PDGF-BB;
- 2 to 100 pg/ml KGF;
- 1 to 30 ng/ml TGF-β1, or 0.4 to 12 ng/ml TGF-β1;
- 30 to 222.5 pg/ml VEGF, or 12 to 89 pg/ml VEGF;
- 60 to 5000 ng/ml PEDF, or 24 to 2000 ng/ml PEDF; and
- 1750 to 2600 mg/dl albumin, or 700 to 1040 mg/dl albumin.

7. Product according to any one of the preceding claims, comprising
- 50 to 250 ng/ml IGF-1, or 20 to 100 ng/ml IGF-1.

8. Product according to any one of claims 1 to 7 for use in medicine.

9. Product according to any one of claims 1 to 7 for use in the treatment of dry eye disease.

10. Product according to claim 8 for use in medicine, or product according to claim 9 for use in the treatment of dry eye disease, wherein the human serum composition is derived from allogeneic blood.

11. Ophthalmic composition, such as eye drops or gels, comprising the product according to any one of claims 1 to 7.

12. Method for manufacturing a product, comprising the steps of
- providing human allogeneic blood volume portions from a multitude of donors,
- selecting volume portions from at least two donors,
- deriving serum from the selected volume portions to obtain serum portions,
- pooling the selected serum portions to obtain pooled serum, wherein optionally the selected serum portions are from donors all having an age of 45 years or less;
- obtaining an intermediate product from the pooled serum, the intermediate product comprising 100 ng/ml IGF-1 or more; and
- obtaining the human serum product according to any one of claims 1 to 6 from the intermediate product.

13. Method according to claim 11, wherein
(i) the intermediate product is serum, wherein obtaining the intermediate product comprises preparing serum from the human allogeneic blood; or
(ii) the intermediate product is a retentate of an ultrafiltration process, wherein obtaining the intermediate product comprises preparing serum from the human allogeneic blood, ultrafiltrating the serum to obtain a filtrate and a retentate, and selecting the retentate.

14. Method according to claim 13, wherein the ultrafiltration process of step (ii) is carried with a molecular cut-off membrane of at least 20 kDa, or at least 30 kDa, or at least 50 kDa, or at least 100 kDa, or wherein the step of obtaining the human serum product further comprises diluting the intermediate product.

15. Method according to any one of claims 12 to 14,
wherein the intermediate product comprises less than 140 mg/dl monosaccharides, and/or 10 to 200 mg/dl total cholesterol, wherein total cholesterol is the sum of LDL (low-density lipoprotein), HDL (high-density lipoprotein) and VLDL (very low-density lipoprotein).

## Patentansprüche

1. Produkt von Humanserum von mehreren Spendern umfassend insulinähnliche Wachstumsfaktoren (IGFs) gebunden in makromolekularen Komplexen mit einer Molekülgröße von 30 kDa oder höher, wobei das Produkt 20 ng/ml IGF-1 oder mehr umfasst,
das Produkt weniger als 70 mg/dl Monosaccharide umfasst,
wobei das Produkt gegebenenfalls eine isotonische Lösung oder ein isotonisches Gel ist.

2. Produkt gemäß Anspruch 1, wobei das Produkt weniger als 40 mg/dl Gesamtcholesterin umfasst, wobei Gesamtcholesterin die Summe von LDL (Lipoprotein niedriger Dichte) und HDL (Lipoprotein hoher Dichte) ist.

3. Produkt gemäß Anspruch 1 oder Anspruch 2, wobei das Produkt eine Wundverschluss-Leistung in einem *in vitro* Wundverschluss-Test von 50 % oder höher aufweist, normalisiert auf eine Verletzungsfläche, bei 35 Stunden, wobei der *in vitro* Wundverschluss-Test die folgenden Schritte umfasst
(a) Bereitstellen einer in vitro Zellkultur umfassend HCE-T Zellen,
(b) Verwenden einer Mikroverletzung, z.B. einer Kratzwunde auf der (Mono)schicht von HCE-T Zellen,
(c) Aufbringen des Produkts von Humanserum,
(d) Überwachen der in vitro Zellkultur, z.B. über Lichtmikroskopie, und
(e) Quantifizieren des zeitabhängigen Wundverschlusses als Wundverschluss-Leistung durch Berechnen des prozentualen Unterschieds bei der Wundfläche zu verschiedenen Zeitpunkten.

4. Produkt gemäß einem der vorhergehenden Ansprüche, wobei das Produkt eine entzündungshemmende Leistung in einem Entzündungstest aufweist, wobei die entzündungshemmende Leistung auf einen standardisierten Ausdruck von LPSinduziertem TNF-α (als ein Referenzbasiswert) normalisiert ist, wobei die entzündungshemmende Leistung 50 % oder mehr beträgt,
wobei die entzündungshemmende Leistung in einem Entzündungstest bestimmt wird durch
- Bereitstellen von Makrophagen von mindestens fünf Spendern,
- Kombinieren der isolierten Makrophagen;
- Stimulieren der kombinierten Makrophagen mit LPS (Lipopolysaccharid), um TNF-α Sekretion zu induzieren, was als ein Referenzwert für Entzündung dient;
- Testen des Produkts von Humanserum in dreifacher Ausführung, wenn die kombinierten Makrophagen mit LPS stimuliert werden, was als ein Wert für entzündungshemmende Leistung dient;
- Testen von Prednisolon, bevorzugt Prednisolonacetat (1,0 Gew.-%), wenn die kombinierten Makrophagen mit LPS stimuliert werden, was als ein Wert für 100 % entzündungshemmende Leistung dient, d.h. als Positivkontrolle dient;
- Testen von Medium, wenn die kombinierten Makrophagen mit LPS stimuliert werden, was als ein Wert für 0 % entzündungshemmende Leistung dient, d.h. als eine Negativkontrolle dient; und
- Quantifizieren einer entzündungshemmenden Leistung für das Produkt von Humanserum in %, normalisiert in Bezug auf die Positivkontrolle und die Negativkontrolle,
und/oder wobei das Produkt weniger als 0,2 mg/dl C-reaktives Protein (CRP) umfasst.

5. Produkt gemäß einem der vorhergehenden Ansprüche, umfassend einen oder mehr Wachstumsfaktoren ausgewählt aus der Liste von EGF, IGF-BP3, IGF-2, PDGF-BB, Alpha 2-Makroglobulin, KGF, und PEDF.

6. Produkt gemäß einem der vorhergehenden Ansprüche, umfassend einen oder mehr Wachstumsfaktoren in den folgenden Konzentrationsbereichen:
- 50 bis 500 pg/ml EGF, oder 20 bis 200 pg/ml EGF,
- 650 bis 4000 ng/ml IGF-BP3, oder 260 bis 1600 ng/ml IGF-BP3;
- 450 bis 6000 ng/ml ALS, oder 180 bis 2400 ng/ml ALS,
- 0,37 bis 1,49 g/l Alpha 2-Makroglobulin, oder 0,15 bis 0,6 g/l Alpha 2-Makroglobulin;
- 471 bis 3683 pg/ml PDGF-BB, oder 188,4 bis 1473,2 pg/ml PDGF-BB,
- 2 bis 100 pg/ml KGF;
- 1 bis 30 ng/ml TGF-β1, oder 0,4 bis 12 ng/ml TGF-β1;
- 30 bis 222,5 pg/ml VEGF, oder 12 bis 89 pg/ml VEGF;
- 60 bis 5000 ng/ml PEDF, oder 24 bis 2000 ng/ml PEDF, und
- 1750 bis 2600 mg/dl Albumin, oder 700 bis 1040 mg/dl Albumin.

7. Produkt gemäß einem der vorhergehenden Ansprüche, umfassend
- 50 bis 250 ng/ml IGF-1, oder 20 bis 100 ng/ml IGF-1.

8. Produkt gemäß einem der Ansprüche 1 bis 7 zur Verwendung in Medizin.

9. Produkt gemäß einem der Ansprüche 1 bis 7 zur Verwendung in der Behandlung der Krankheit des trockenen Auges.

10. Produkt gemäß Anspruch 8 zur Verwendung in Medizin, oder Produkt gemäß Anspruch 9 zur Verwendung in der Behandlung der Krankheit des trockenen Auges, wobei die Humanserum-Zusammensetzung von allogenem Blut abgeleitet ist.

11. Ophthalmische Zusammensetzung, wie Augentropfen oder -gele, umfassend das Produkt gemäß einem der Ansprüche 1 bis 7.

12. Verfahren zur Herstellung eines Produkts, umfassend die Schritte von
- Bereitstellen von Volumenanteilen von humanem allogenem Blut von einer Mehrzahl von Spendern,
- Auswählen von Volumenanteilen von mindestens zwei Spendern,
- Ableiten von Serum von den ausgewählten Volumenanteilen, wobei Serumanteile erhalten werden,
- Vereinigen der ausgewählten Serumanteile, wobei vereinigtes Serum erhalten wird, wobei gegebenenfalls die ausgewählten Serumanteile von Spendern stammen, welche alle ein Alter von 45 Jahren oder niedriger aufweisen;
- Erhalten eines Zwischenprodukts von dem vereinigten Serum, wobei das Zwischenprodukt 100 ng/ml IGF-1 oder mehr umfasst, und
- Erhalten des Produkts von Humanserum gemäß einem der Ansprüche 1 bis 6 von dem Zwischenprodukt.

13. Verfahren gemäß Anspruch 12, wobei
(i) das Zwischenprodukt Serum ist, wobei Erhalten des Zwischenprodukts Herstellen von Serum aus dem humanen allogenen Blut umfasst, oder
(ii) das Zwischenprodukt ein Retentat von einem Ultrafiltrationsverfahren ist, wobei Erhalten des Zwischenprodukts Herstellen von Serum aus dem humanen allogenen Blut, Ultrafiltrieren des Serums, um ein Filtrat und ein Retentat zu erhalten, und Auswählen des Retentats umfasst.

14. Verfahren gemäß Anspruch 13, wobei das Ultrafiltrationsverfahren von Schritt (ii) mit einer molekularer Cutoff-Membran von mindestens 20 kDa, oder mindestens 30 kDa, oder mindestens 50 kDa, oder mindestens 100 kDa durchgeführt wird, oder wobei der Schritt von Erhalten des Produkts von Humanserum ferner Verdünnen des Zwischenprodukts umfasst.

15. Verfahren gemäß einem der Ansprüche 12 bis 14,
wobei das Zwischenprodukt weniger als 140 mg/dl Monosaccharide, und/oder 10 bis 200 mg/dl Gesamtcholesterin umfasst, wobei Gesamtcholesterin die Summe von LDL (Lipoprotein niedriger Dichte), HDL (Lipoprotein hoher Dichte) und VLDL (Lipoprotein sehr niedriger Dichte) ist.

## Revendications

1. Produit sérum humain multi-donneurs comprenant des facteurs de croissance ressemblant à l'insuline (IGFs) liés dans des complexes macromoléculaires d'une taille moléculaire supérieure ou égale à 30 kDa, le produit comprenant 20 ng/ml d'IGF-1 ou davantage,
le produit comprenant moins de 70 mg/dl de monosaccharides,
dans lequel le produit est optionnellement une solution isotonique ou un gel isotonique.

2. Produit selon la revendication 1, dans lequel le produit comprend moins de 40 mg/dl de cholestérol total, dans lequel le cholestérol total est la somme des LDL (lipoprotéines de basse densité) et des HDL (lipoprotéines de haute densité).

3. Procédé selon la revendication 1 ou la revendication 2, dans lequel le produit présente des performances de fermeture de plaie dans un test de fermeture de plaie in vitro supérieures ou égales à 50 %, normalisées à une zone de blessure, à 35 heures, dans lequel le test de fermeture de plaie in vitro comprend les étapes
(a) fournir une culture de cellules in vitro comprenant des cellules HCE-T,
(b) utiliser une micro-blessure, par exemple une éraflure sur la (mono)couche de cellules HCE-T,
(c) appliquer le produit sérum humain,
(d) surveiller la culture de cellules in vitro, par exemple par microscopie lumineuse et
(e) quantifier temporellement la fermeture de plaie sous forme de performances de fermeture de plaie en calculant la différence de pourcentage dans la zone de plaie à différents instants.

4. Produit selon l'une quelconque des revendications précédentes, dans lequel le produit présente des performances anti-inflammatoires dans un test d'inflammation, dans lequel les performances anti-inflammatoires sont normalisées sous la forme d'une expression standardisée des TNF-α induits par LPS (en tant que valeur de base de référence), dans lequel les performances anti-inflammatoires sont supérieures ou égales à 50 %,
dans lequel les performances anti-inflammatoires dans un test d'inflammation sont déterminées
- en fournissant des macrophages en provenance d'au moins cinq donneurs ;
- en combinant les macrophages isolés ;
- en stimulant les macrophages combinés par des LPS (lipopolysaccharides) pour induire une sécrétion de TNF-α qui sert de valeur de référence pour l'inflammation ;
- en testant le produit sérum humain en trois exemplaires lors de la stimulation des macrophages combinés avec des LPS, qui fournit une valeur de performances anti-inflammatoires ;
- en testant la prednisolone, de préférence l'acétate de prednisolone (1,0 % en poids), lors de la stimulation des macrophages combinés par des LPS, qui fournit une valeur pour des performances anti-inflammatoires de 100 %, c'est-à-dire servant de contrôle positif ;
- en testant le milieu lors de la stimulation des macrophages combinés avec des LPS, qui fournit une valeur pour des performance anti-inflammatoires de 0 %, c'est-à-dire servant de contrôle négatif ; et
- en quantifiant les performance anti-inflammatoires pour le produit sérum humain en % normalisés en relation avec le contrôle positif et le contrôle négatif,
et/ou dans lequel le produit comprend moins de 0,2 mg/dl de protéine C-réactive (CRP).

5. Produit selon l'une quelconque des revendications précédentes, comprenant un ou plusieurs facteurs de croissance choisis dans la liste comprenant les EGF, IGF-BP3, IGF-2, PDGF-BB, alpha 2-macroglobuline, KGF et PEDF.

6. Produit selon l'une quelconque des revendications précédentes, comprenant un ou plusieurs facteurs de croissance compris dans les plages de concentrations suivantes :
- de 50 à 500 pg/ml d'EGF ou de 20 à 200 pg/ml d'EGF ;
- de 650 à 4000 ng/ml d'IGF-BP3 ou de 260 à 1600 ng/ml d'IGF-BP3 ;
- de 450 à 6000 ng/ml d'ALS ou de 180 à 2400 ng/ml d'ALS ;
- de 0,37 à 1,49 g/I d' alpha 2-macroglobuline ou de 0,15 à 0,6 g/I d'alpha 2-macroglobuline ;
- de 471 à 3683 pg/ml de PDGF-BB ou de 188,4 à 1473,2 pg/ml de PDGF-BB ;
- de 2 à 100 pg/ml de KGF ;
- de 1 à 30 ng/ml de TGF-β1 ou de 0,4 à 12 ng/ml de TGF-β1 ;
- de 30 à 222,5 pg/ml de VEGF ou de 12 à 89 pg/ml de VEGF ;
- de 60 à 5000 ng/ml de PEDF ou de 24 à 2000 ng/ml de PEDF ; et
- de 1750 à 2600 mg/dl d'albumine ou de 700 à 1040 mg/dl d'albumine.

7. Produit selon l'une quelconque des revendications précédentes, comprenant
- de 50 à 250 ng/ml d'IGF-1 ou de 20 à 100 ng/ml d'IGF-1.

8. Produit selon l'une quelconque des revendications 1 à 7 destiné à être utilisé dans un médicament.

9. Produit selon l'une quelconque des revendications 1 à 7 destiné à être utilisé dans le traitement de la maladie de l'oeil sec.

10. Procédé selon la revendication 8 destiné à être utilisé dans un médicament ou produit selon la revendication 9 destiné à être utilisé dans le traitement de la maladie de l'oeil sec, dans lequel la composition du sérum humain est dérivée de sang allogénique.

11. Composition ophtalmique, telle que du collyre ou du gel pour les yeux, comprenant le produit selon l'une quelconque des revendications 1 à 7

12. Procédé de fabrication d'un produit, comprenant les étapes
- fournir des parties de volume de sang allogénique humain en provenance d'une multitude de donneurs,
- choisir des parties de volume en provenance d'au moins deux donneurs,
- dériver du sérum en provenance des parties de volume choisies pour obtenir des parties de sérum,
- mettre en commun les parties de sérum choisies pour obtenir un sérum combiné, dans lequel optionnellement les parties de sérum choisies proviennent de donneurs tous âgés de 45 ans ou moins ;
- obtenir un produit intermédiaire à partir du sérum combiné, le produit intermédiaire comprenant 100 ng/ml d'IGF-1 ou davantage ; et
- obtenir le produit sérum humain selon l'une quelconque des revendications 1 à 6 à partir du produit intermédiaire.

13. Procédé selon la revendication 11, dans lequel
(i) le produit intermédiaire est un sérum, dans lequel l'obtention du produit intermédiaire comprend la préparation de sérum à partir du sang allogénique humain ; ou
(ii) le produit intermédiaire est un rétentat d'un processus d'ultrafiltration, dans lequel l'obtention du produit intermédiaire comprend la préparation de sérum à partir du sang allogénique humain, l'ultrafiltration du sérum pour obtenir un filtrat et un rétentat et la sélection du rétentat.

14. Procédé selon la revendication 13, dans lequel le processus d'ultrafiltration de l'étape (ii) est réalisé avec une membrane présentant un seuil de rétention d'au moins 20 kDa ou d'au moins 30 kDa ou d'au moins 50 kDa ou d'au moins 100 kDa, ou dans lequel l'étape d'obtention du produit sérum humain comprend en outre la dilution du produit intermédiaire.

15. Procédé selon l'une quelconque des revendications 12 à 14,
dans lequel le produit intermédiaire comprend moins de 140 mg/dl de monosaccharides et/ou de 10 à 200 mg/dl de cholestérol total, dans lequel le cholestérol total est la somme des LDL (lipoprotéines de basse densité), des HDL (lipoprotéines de haute densité) et des VLDL (lipoprotéines de très basse densité).
